(19) Europäisches Patentamt
European Patent Office
Office européen des brevets

(11) EP 1 647 275 A1

(12) EUROPEAN PATENT APPLICATION
published in accordance with Art. 158(3) EPC

(43) Date of publication:
**19.04.2006 Bulletin 2006/16**

(21) Application number: **04747894.6**

(22) Date of filing: **16.07.2004**

(51) Int Cl.:
*A61K 31/522* (2000.01) *A61K 9/20* (1974.07)
*A61K 47/38* (1990.01) *A61P 25/16* (2000.01)
*A61P 25/24* (2000.01) *A61P 25/28* (2000.01)

(86) International application number:
**PCT/JP2004/010530**

(87) International publication number:
**WO 2005/007167 (27.01.2005 Gazette 2005/04)**

(84) Designated Contracting States:
**AT BE BG CH CY CZ DE DK EE ES FI FR GB GR HU IE IT LI LU MC NL PL PT RO SE SI SK TR**

(30) Priority: **17.07.2003 JP 2003198434**

(71) Applicant: **KYOWA HAKKO KOGYO CO., LTD.**
**Chiyoda-ku,**
**Tokyo 100-8185 (JP)**

(72) Inventors:
- **UCHIDA, Akihiro,**
**c/o Fuji Plant, Kyowa Hakko Kogyo**
**Sunto-gun, Shizuoka 411-8731 (JP)**
- **ISHIKAWA, Yasuhiro**
**Pharmaceutical Research Center**
**Nagaizumi-cho,Sunto-gun,Shizuoka4118731 (JP)**
- **UENO, Yasuhiko**
**c/o Fuji Plant,Kyowa Hakko Kogyo Co**
**Sunto-gun, Shizuoka 411-8731 (JP)**
- **KAJI, Kiichiro**
**c/oBioFrontier Laboratories, Kyowa**
**Machida-shi, Tokyo 194-8533 (JP)**
- **TOTTORI, Tsuneaki**
**Osaka 565-0851 (JP)**

(74) Representative: **Vossius & Partner**
**Siebertstrasse 4**
**81675 München (DE)**

# (54) SOLID PHARMACEUTICAL PREPARATION

(57)

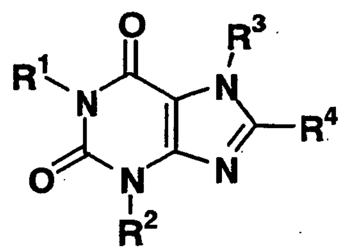
(I)

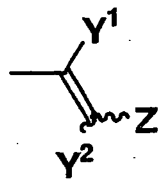
(II)

(wherein $R^1$, $R^2$ and $R^3$ may be the same or different and each represents a hydrogen atom, lower alkyl, lower alkenyl or lower alkynyl; and $R^4$ represents cycloalkyl, $-(CH_2)_n-R^5$ or the above formula (II))

The present invention provides a solid formulation comprising a xanthine derivative represented by the above formula (I) or a pharmaceutically acceptable salt thereof, and microcrystalline cellulose, which possesses excellent pharmaceutical properties, for example, in hardness, disintegration property, dissolution property, stability or the like.

EP 1 647 275 A1

FIG.1

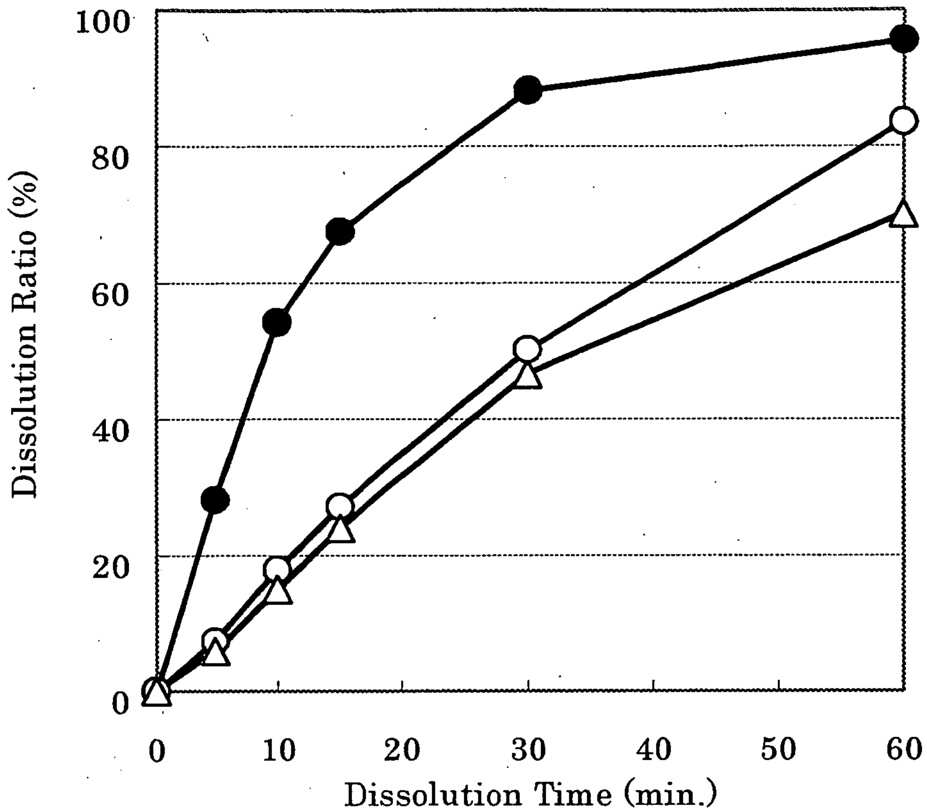
100
80
60
40
20
0
Dissolution Ratio (%)
0
10
20
30
40
50
60
Dissolution Time (min.)

## Description

Technical Field

The present invention relates to a solid formulation comprising a xanthine derivative or a pharmaceutically acceptable salt thereof.

Background Art

It is known that xanthine derivatives represented by formula (I) described below [referred to as Compound (I) herein below] or pharmaceutically acceptable salts thereof shows adenosine $A_2$ receptors antagonistic activity and are therefore useful for the therapeutic treatment of various diseases induced by hyperactivity of adenosine $A_2$ receptor such as Parkinson disease, senile dementia, depression or the like (e.g. EP 0 590 919).

Additionally, for example, tablets comprising lactose, potato starch, hydroxypropylcellulose and (E)-8-(3,4-dimethoxystyryl)-1,3-diethyl-7-methyl-3,7-dihydro -1H-purine-2,6-dione (referred to as Compound 1 hereinbelow), and the like have been known (Japanese Published Unexamined Patent Application No. 211856/94). However, solid formulations comprising Compound (I) or a pharmaceutically acceptable salt thereof having such general composition as described in Japanese Published Unexamined Patent Application No. 211-856/94 have the following problems as to pharmaceutical properties: (a) the hardness is insufficient; (b) the disintegration time is long; (c) the dissolution is likely to be delayed; (d) the stability is poor, and so on. The development of a solid formulation comprising Compound (I) or a pharmaceutically acceptable salt thereof, which have overcome the problems, is desired.

Disclosure of the Invention

The object of the present invention is to provide a solid formulation comprising Compound (I) or a pharmaceutically acceptable salt thereof, which possesses excellent pharmaceutical properties (for example, in hardness, disintegration property, dissolution property, stability or the like).

The invention relates to the following (1) to (22).

(1) A solid formulation comprising a xanthine derivative represented by formula (I)

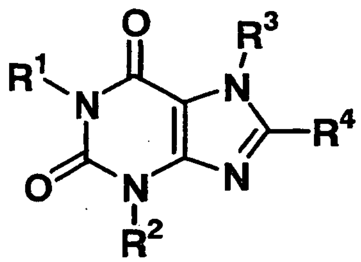

(I)

[wherein $R^1$, $R^2$ and $R^3$ may be the same or different and each represents a hydrogen atom, lower alkyl, lower alkenyl or lower alkynyl; and $R^4$ represents cycloalkyl, $-(CH_2)_n-R^5$ (in which $R^5$ represents substituted or unsubstituted aryl or a substituted or unsubstituted heterocyclic group; and n represents an integer of 0 to 4) or formula (II)

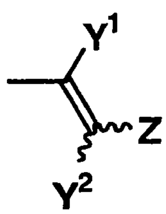

(II)

(in which $Y^1$ and $Y^2$ maybe the same or different and each represents a hydrogen atom, halogen or lower alkyl; and Z represents substituted or unsubstituted aryl or a substituted or unsubstituted heterocyclic group)] or a pharmaceutically acceptable salt thereof, and microcrystalline cellulose.

(2) The solid formulation according to the above (1), wherein $R^4$ is formula (II)

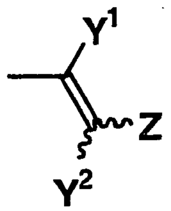

(II)

(in which $Y^1$, $Y^2$ and Z have the same meanings as described above, respectively).

(3) The solid formulation according to the above (2), wherein $Y^1$ and $Y^2$ each are a hydrogen atom.

(4) The solid formulation according to the above (2) or (3), wherein Z is substituted or unsubstituted aryl or formula (III)

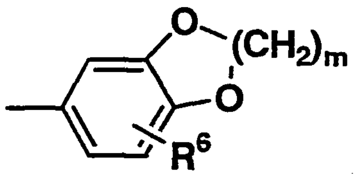

(III)

(in which $R^6$ represents a hydrogen atom, hydroxy, lower alkyl, lower alkoxy, halogen, nitro or amino; and m represents an integer of 1 to 3).

(5) A solid formulation comprising (E)-8-(3,4-dimethoxystyryl)-1,3-diethyl-7-methyl-3,7-dihydro -1H-purine-2,6-dione represented by formula (IA)

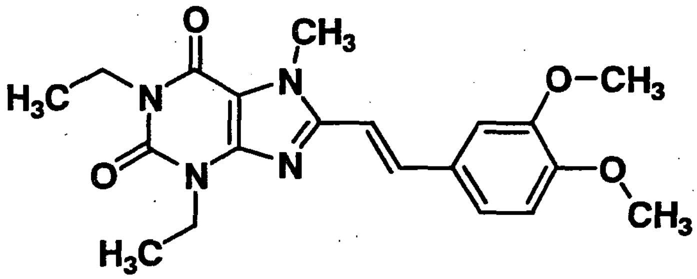

(IA)

or a pharmaceutically acceptable salt thereof, and microcrystalline cellulose.

(6) The solid formulation according to any one of the above (1) to (5), which contains a sugar.

(7) The solid formulation according to the above (6), wherein the sugar content is 1.0 to 9.0 parts by weight per 1.0 part by weight of microcrystalline cellulose.

(8) The solid formulation according to the above (6), wherein the sugar content is 1.0 to 5.0 parts by weight per 1.0 part by weight of microcrystalline cellulose.

(9) The solid formulation according to the above (6), wherein the sugar content is 1.5 to 3.0 parts by weight per 1.0 part by weight of microcrystalline cellulose.

(10) The solid formulation according to any one of the above (6) to (9), wherein the sugar is lactose, sucrose, glucose, cyclodextrin or mannitol.

(11) The solid formulation according to any one of the above (6) to (9), wherein the sugar is lactose.

(12) The solid formulation according to any one of the above (1) to (11), which contains a binder.

(13) The solid formulation according to the above (12), wherein the binder is hydroxypropylcellulose, hydroxypropylmethylcellulose, polyvinylpyrrolidone or polyvinyl alcohol.

(14) The solid formulation according to the above (12), wherein the binder is polyvinyl alcohol.

(15) The solid formulation according to any one of the above (12) to (14), wherein the binder content is 0.1 to 10.0 % of the total weight of the solid formulation.

(16) The solid formulation according to any one of the above (1) to (15), which contains a disintegrator.

(17) The solid formulation according to the above (16), wherein the disintegrator is crospovidone, croscarmellose sodium, low substituted hydroxypropylcellulose, carmellose calcium or sodium starch glycolate.

(18) The solid formulation according to the above (16), wherein the disintegrator is crospovidone.

(19) The solid formulation according to any one of the above (16) to (18), wherein the disintegrator content is 0.5 to 20.0 % of the total weight of the solid formulation.
(20) The solid formulation according to any one of the above (1) to (19), wherein a dosage form of the solid formulation is a tablet.
(21) The solid formulation according to any one of the above (1) to (20), which is a film-coated solid formulation.
(22) A method for stabilizing a xanthine derivative represented by formula (I)

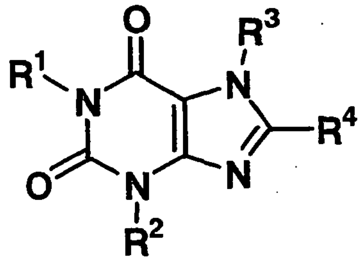

(I)

(wherein $R^1$, $R^2$, $R^3$ and $R^4$ have the same meanings as described above, respectively) or a pharmaceutically acceptable salt thereof in a solid formulation comprising the xanthine derivative or the pharmaceutically acceptable salt thereof, which comprises allowing microcrystalline cellulose to exist in the solid formulation.

In the definition of each group of formula (I):

Examples of the lower alkyl and the lower alkyl moiety in the lower alkoxy include straight-chain or branched alkyl groups having 1 to 6 carbon atoms and specifically include methyl, ethyl, propyl, isopropyl, butyl, isobutyl, sec-butyl, tert-butyl, pentyl, neopentyl, hexyl and the like.
Examples of the lower alkenyl include straight-chain or branched alkenyl groups having 2 to 6 carbon atoms and specifically include vinyl, allyl, methacryl, crotyl, 3-butenyl, 2-pentenyl, 4-pentenyl, 2-hexenyl, 5-hexenyl and the like.
Examples of the lower alkynyl include straight-chain or branched alkynyl groups having 2 to 6 carbon atoms and specifically include ethynyl, propargyl, 2-butynyl, 3-butynyl, 2-pentynyl, 4-pentynyl, 2-hexynyl, 5-hexynyl, 4-methyl-2-pentynyl and the like.
Examples of the cycloalkyl include cycloalkyl groups having 3 to 8 carbon atoms and specifically include cyclopropyl, cyclobutyl, cyclopentyl, cyclohexyl, cycloheptyl, cyclooctyl and the like.

The halogen means fluorine, chlorine, bromine and iodine atoms.
Examples of the aryl include aryl groups having 6 to 14 carbon atoms and specifically include phenyl, naphthyl, anthryl and the like.
Examples of the heterocyclic group include 5-membered or 6-membered monocyclic heterocyclic groups containing at least one atom selected from a nitrogen atom, an oxygen.atom and a sulfur atom and bicyclic or tricyclic condensed heterocyclic groups containing at least one atom selected from a nitrogen atom, an oxygen atom and a sulfur atom in which 3- to 8-membered rings are condensed and specifically include furyl, thienyl, pyrrolyl, pyranyl, thiopyranyl, pyridyl, pyrimidinyl, triazinyl, purinyl, pyrazinyl, pyridazinyl, benzimidazolyl, 2-oxobenzimidazolyl, benzotriazolyl, benzofuryl, benzothienyl, benzoxazolyl, benzothiazolyl, 1,3-benzodioxolyl, 1,4-benzodioxanyl, 3,4-dihydro-2H-1,5-benzodioxepinyl, indazolyl, indolyl, isoindolyl, quinolyl, isoquinolyl,phthalazinyl,naphthyridinyl, quinoxalinyl, pyrazolyl, quinazolinyl, cinnolinyl, triazolyl, tetrazolyl, imidazolyl, oxazolyl, isoxazolyl, thiazolyl, isothiazolyl, dihydroisoquinolyl, tetrahydroquinolyl, dihydrobenzopyranyl and the like.
The substituted aryl and the substituted heterocyclic group each have, for example, 1 to 3 substituents which may be the same or different, and examples of the substituents include lower alkyl, lower alkenyl, lower alkynyl, hydroxy, substituted or unsubstituted lower alkoxy, halogen, nitro, amino, lower alkylamino, di-lower alkylamino, trifluoromethyl, trifluoromethoxy, aralkyl, aralkyloxy, aryl, aryloxy, lower alkanoyl, lower alkanoyloxy, aroyl, aroyloxy, arylalkanoyloxy, carboxy, lower alkoxycarbonyl, lower alkylcarbamoyl, di-lower alkylcarbamoyl, sulfo, lower alkoxysulfonyl, lower alkylsulfamoyl, di-lower alkylsulfamoyl and the like.
In the examples of the substituents mentioned above:
The lower alkyl moieties of the lower alkyl, the lower alkoxy, the lower alkylamino, the di-lower alkylamino, the lower alkanoyl, the lower alkanoyloxy, the lower alkoxycarbonyl, the lower alkylcarbamoyl, the di-lower alkylcarbamoyl, the lower alkoxysulfonyl, the lower alkylsulfamoyl and the di-lower alkylsulfamoyl have the same meaning as the lower alkyl described above; the halogen, the lower alkenyl and the lower alkynyl each have the same meanings as described

above; two lower alkyl moieties of the di-lower alkylamino, the di-lower alkylcarbamoyl and the di-lower alkylsulfamoyl each may be the same or different; the aryl moieties of the aryl and the aryloxy have the same meaning as the aryl described above; the aralkyl moieties of the aralkyl and the aralkyloxy include benzyl, phenethyl and the like; the aroyl moieties of the aroyl and the aroyloxy include benzoyl, naphthoyl and the like; and the arylalkyl moiety of the arylalkanoyloxy includes benzyl, phenethyl and the like.

The substituted lower alkoxy has, for example, 1 to 3 substituents which may be the same or different, and examples of the substituents include hydroxy, lower alkoxy, halogen, amino, azido, carboxy, lower alkoxycarbonyl and the like; herein, the lower alkyl moieties of the lower alkoxy and the lower alkoxycarbonyl have the same meaning as the lower alkyl described above; and the halogen has the same meaning as described above.

Examples of the pharmaceutically acceptable salts of Compound (I) include pharmaceutically acceptable acid addition salts, metal salts, ammonium salt, organic amine addition salts, amino acid addition salts and the like.

Examples of the pharmaceutically acceptable acid addition salts of Compound (I) include inorganic acid salts such as a hydrochloride, a sulfate and a phosphate, and organic acid salts such as an acetate, a maleate, a fumarate, a tartrate, a citrate and a methanesulfonate; examples of the pharmaceutically acceptable metal salts include alkali metal salts such as a sodium salt and a potassium salt, alkaline earth metal salts such as a magnesium salt and a calcium salt, an aluminium salt, a zinc salt, and the like; the pharmaceutically acceptable ammonium salts include ammonium, tetramethylammonium and the like; examples of the pharmaceutically acceptable organic amine addition salts include addition salts with morpholine, piperidine or the like; and examples of the pharmaceutically acceptable amino acid addition salts include addition salts with lysine, glycine, phenylalanine or the like.

In the solid formulation of the present invention:

(i) A method for production of Compound (I) or a pharmaceutically acceptable salt thereof, for example (E)-8-(3,4-dimethoxystyryl)-1,3-diethyl-7-methyl-3,7-dihydro -1H-purine-2, 6-dione (Compound1) represented by the formula (IA)

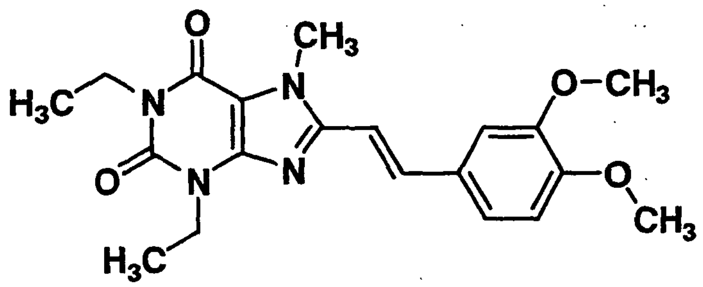

(IA)

or a pharmaceutically acceptable salt thereof, is not particularly limited, but Compound (I) or a pharmaceutically acceptable salt thereof canbe obtained by methods described in Japanese Published Unexamined Patent Application No. 211856/94, EP 0 590 919, Japanese Published Unexamined Patent Application No. 040652/97 or the like, or modified methods thereof.

Compound (I) or a pharmaceutically acceptable salt thereof used for the solid formulation of the present invention is not particularly limited, so long as it is a solid such as a powder, crystals or a mass. Examples includes the crystalline Compound (I) having a crystallinity of 20 % or more, or a pharmaceutically acceptable salt thereof. Among them, the crystalline Compound (I) having a crystallinity of 30 % or more, or a pharmaceutically acceptable salt thereof is preferable, and the crystalline Compound (I) a crystallinity of 40 % or more or a pharmaceutically acceptable salt thereof is more preferable. The crystallinity of Compound (I) or a pharmaceutically acceptable salt thereof means the relative amount of "the crystalline Compound (I) or a pharmaceutically acceptable salt thereof" in "Compound (I) or a pharmaceutically acceptable salt thereof", and canbe calculated from the following formula.

$$\text{The Crystallinity (\%)} = \frac{\text{Amount of " the crystalline Compound (I) or a pharmaceutically acceptable salt thereof "}}{\text{Amount of " Compound (I) or a pharmaceutically acceptable salt thereof "}} \text{X100}$$

The amount of "Compound (I) or a pharmaceutically acceptable salt thereof" means the total amount of "the crystalline

Compound (I) or a pharmaceutically acceptable salt thereof" and "an amorphous Compound (I) or a pharmaceutically acceptable salt thereof". The crystallinity can be calculated by measuring the integral intensity of the diffraction peak at a specific angle of diffraction 2θ, for example by a powder X-ray diffractmeter (e.g., JDX8030; manufactured by JEOL Ltd.), that is the crystallinity can be determined as the ratio of the integral intensity of the diffraction peak of a sample measured to the integral intensity of the diffraction peak of a standard sample. The proportion of "the crystalline Compound (I) or a pharmaceutically acceptable salt thereof" in the standard sample is 100 % (the crystallinity of 100 %). These can be obtained by methods described in Japanese Published Unexamined Patent Application No. 211856/94, EP 0 590 919, Japanese Published Unexamined Patent Application No. 040652/97 or the like, or modified methods thereof.

In addition, among the crystalline Compound (I) having the crystallinity of 20 % or more, or a pharmaceutically acceptable salt thereof, such compound that having an average particle size of 50 μm or less, or a pharmaceutically acceptable salt thereof is preferable. Particularly, the crystalline Compound (I) having an average particle size of 0.5 to 20 μm and the crystallinity of 20 % or more, or a pharmaceutically acceptable salt thereof is more preferable. In this connection, the average particle size can be measured, by using, for example, a laser diffraction / scattering particle size distribution analyzer (e.g., MASTERSIZER 2000 Ver. 2.00 J; manufactured by MALVERN instruments) or an image analyzer (e.g., LUZEX® AP; manufactured by NIRECO Co.), etc., and can be calculated from the mean of the particle size distribution. These can be prepared by pulverization and/or sieving the crystalline Compound (I) having the crystallinity of 20 % or more or a pharmaceutically acceptable salt thereof, which is obtained by methods described in Japanese Published Unexamined Patent Application No. 211856/94, EP 0 590 919, Japanese Published Unexamined Patent Application No. 040652/97 or the like, or modified method thereof. The pulverization and the sieving may be appropriately carried out in combination several times. The pulverization can be carried out by a pulverizer generally used, such as a mortar, Mechanomill® (manufactured by Okada Seiko Co., Ltd.), and a jet mill. In the pulverization, the pulverization conditions, such as the rotational speed of the pulverizer; the feed rate of the crystalline Compound (I) having a crystallinity of 20 % or more, or a pharmaceutically acceptable salt thereof; the time required for pulverization; and the like, are appropriately controlled to obtain the crystalline Compound (I) having a desired average particle size and/or a desired crystallinity, or a pharmaceutically acceptable salt thereof. Among them, the pulverization by the jet mill is preferable, and the crystalline Compound (I) having a crystallinity of 20 % or more, or a pharmaceutically acceptable salt thereof can be pulverized, by feeding the crystalline Compound (I) having a crystallinity of 20 % or more, or a pharmaceutically acceptable salt thereof, at a rate of 10 to 1,000 g/min and under pressure of 0.01 to 1.0 MPa.

The amount of Compound (I) or a pharmaceutically acceptable salt thereof in the solid formulation of the present invention is not particularly limited. In case of Compound 1, for example, the proportion of Compound 1 in the solid formulation is preferably 1 to 50 %, more preferably 2 to 30 %, still more preferably 5 to 20 % of the total weight of the solid formulation.

(ii) The microcrystalline cellulose used in the present invention is not particularly limited, so long as it is generally used in formulations for oral administration. Examples of the microcrystalline cellulose include microcrystalline cellulose and powdered cellulose, which are commercially available.

The microcrystalline cellulose content in the solid formulation of the present invention is not particularly limited, but is preferably 1 to 75 %, more preferably 5 to 50 %, still more preferably 10 to 30 % of the total weight of the solid formulation.

The solid formulation of the present invention may contain additives, which are generally used in formulations for oral administration, such as a vehicle (e.g., starch, a sugar or the like), a binder, a disintegrator or the like. Among the vehicles, a sugar is preferable.

(iii) The sugar used in the present invention is not particularly limited, so long as it is generally used in formulations for oral administration. Examples of the sugar include lactose, sucrose, glucose, cyclodextrin, mannitol and the like. Among them, lactose is preferable.

The sugar content of the solid formulation of the present invention is not particularly limited, but is preferably 1 to 95 %, more preferably 5 to 80 %, still more preferably 20 to 65 % of the total weight of the solid formulation.

When both of a sugar and microcrystalline cellulose are contained in the solid formulation of the present invention, as to the combination ratio of the sugar and microcrystalline cellulose, the sugar content is preferably 1.0 to 9.0 parts by weight, more preferably 1.0 to 5.0 parts by weight, still more preferably 1.5 to 3.0 parts by weight per 1.0 part by weight of microcrystalline cellulose.

(iv) The binder used in the present invention is not particularly limited, so long as it is generally used in formulations for oral administration. Examples of the binder include hydroxypropylcellulose (HPC), hydroxypropylmethylcellulose (HPMC), polyvinylpyrrolidone (PVP), polyvinyl alcohol and the like. Among them, polyvinyl alcohol is preferable. In addition, as the polyvinyl alcohol, polyvinyl alcohol with a polymerization degree of 250 to 5, 000 is preferable. Particularly, polyvinyl alcohol with a polymerization degree of 500 to 5,000 is more preferable.

The binder content of the solid formulation of the present invention is not particularly limited, but is preferably 0.1

to 10.0 %, more preferably 0.5 to 7.0 %, still more preferably 1.0 to 5.0 % of the total weight of the solid formulation.
(v) The disintegrator used in the present invention is not particularly limited, so long as it is generally used in formulations for oral administration. Examples of the disintegrator include sodium alginate, croscarmellose sodium (trade name; Actizol, manufactured by Asahi Kasei Co., Ltd., etc.), sodium starch glycolate (trade name: Exprotab, manufactured by Penwest Pharmaceuticals, etc.), lower substituted hydroxypropylcellulose, carmellose calcium, crospovidone and the like. Among them, crospovidone is preferable.

The disintegrator content of the solid formulation of the present invention is not particularly limited, but is preferably 0.5 to 20.0 %, more preferably 1.0 to 15.0 %, still more preferably 3.0 to 10.0 % of the total weight of the solid formulation.

Examples of a dosage form of the solid formulation of the present invention include tablets, capsules, granules, and the like.

The tablets of the present invention can be prepared by a method generally used in the technical field of pharmaceutics, such as compression molding or the like. Examples of the method include a method which consists of mixing the each component as described above in a blender or the like, and then tableting the resulting mixture as it is, by a tablet-pressing machine, to make the tablets; a method which consists of granulating the each component as described above, and then tableting the resulting granules to form the tablets; and the like. The pressure for tableting can appropriately be selected, for example, within a range of 300 to 3,000 kg/cm$^2$. The size of the tablet is not particularly limited, but the total weight of the tablet is preferably 20 to 3,000 mg per tablet and the particle size of the tablet is preferably 5 to 15 mm.

The granule can be prepared by granulation such as wet granulation, dry granulation or the like. Examples of the wet granulation include fluid-bed granulation or the like, and more specifically include a method, which consist of processes comprising (1) mixing Compound (I) or a pharmaceutically acceptable salt, the sugar and microcrystalline cellulose, and the disintegrator as necessary, (2) spraying a binder solution onto the resulting mixture to prepare a granule, and (3) drying the resulting granule; and the like. Examples of the binder solution for use in spraying include a solution prepared by dissolving the binder in water, ethanol, isopropyl alcohol, a mixture of them or the like, and are most preferably an aqueous solution of the binder. Examples of the dry granulation include a method, which consist of processes comprising (1) forming a flake by a commercially available roller compactor or forming a pellet by a tableting machine, (2) comminuting the resulting flake or pellet by a commercially available a comminuter or a flash-mill to obtain a granule; and the like.

The granules can be prepared, for example, in a manner similar to that in the methods for preparing the granule in the production of the tablets.

The capsules can be prepared, for example, by filling a granule, which is prepared in a manner similar to that in the methods for preparing the granule in the production of the tablets, in capsules.

The solid formulation of the present invention may contain the resulting granules, tablets or the like, which is obtained above, as a core coated with a coated layer (a film forming substance) to form a film layer onto the core. The coated layer is not particularly limited, so long as it is a coating composition containing a coating agent. The coating agent is not particularly limited, so long as it is generally used in formulations for oral administration, and the coating agent which is useful for rapidly releasing an active component from the solid formulation after administration, for example, water soluble, soluble in stomach, soluble in enteric cannel and the like, is preferable. Examples of the coating agent include lactose, HPMC, HPC, hydroxypropylmethyl cellulose phthalate, polyethylene glycol, polyvinyl alcohol, polyvinylacetal diethylaminoacetate, hydroxypropylmethyl cellulose acetate succinate, and methacrylic acid copolymers (trade names: Eudragit E and Eudragit L, manufactured by Röhm Parma Polymers, and the like).

The amount of coating composition to be used is preferably 1 to 20.% by weight, more preferably 3 to 10 % by weight of the core.

Further, the solid formulation of the present invention may be additionally mixed with other additives generally used in formulations for oral administration, such as lubricants, surfactants, plasticizers and the like, if necessary. Examples of the lubricants include magnesium stearate, calcium stearate, talc, light anhydrous silicic acid, hydrated silicon dioxide and the like. Examples of the surfactants include phospholipid, glycerol fatty acid esters (e.g., triacetin and the like), sorbitan fatty acid esters, polyoxyethylene fatty acid esters, polyethylene glycol fatty acid esters, polyoxyethylene hydrogenated castor oil, polyoxyethylene alkyl ether, sucrose fatty acid esters and the like. Examples of the plasticizers include triacetin, vegetable oils, polyethyleneglycol and the like.

Still further, these solid formulations may be prepared by methods generally used in the technical field of pharmaceutics, if necessary, such as a mixing process, a pulverizing process, a sieving process, a granulation process, a milling process, a tableting process, a drying process, a capsule-filling process, a coating process and the like, appropriately in combination.

The method for stabilizing of the present invention can be carried out by the methods for preparing the solid formulation of the present invention described above. Namely, the method can be carried out by preparing a solid

formulation comprising Compound (I) or a pharmaceutically acceptable salt thereof, in which microcrystalline cellulose exists. The solid formulation stabilized by the method for stabilizing of the present invention are not particularly limited as to a dosage form, other additives contained, an amount of each components and the like, so long as it is a solid formulation comprising Compound (I) or a pharmaceutically acceptable salt thereof. Examples of the dosage form, the other additives contained, the amount of each components and the like are the same examples as described above, respectively.

The following test examples specifically illustrate effects of the present invention.

Test Example 1 (Tablet Hardness Test)

Hardness of each of Tablet 1 obtained in Example 1 and Tablets 2 and 3 obtained in Comparative Examples 1 and 2 was measured byatablethardnesstester (PTB-311, manufactured by Pharmatest).

Test Example 2 (Disintegration Test)

According to the method described in Disintegration Test of The Japanese Pharmacopoeia, Fourteenth Edition, Part I, the time required for the disintegration of each of Tablet 1 obtained in Example 1 and Tablets 2 and 3 obtained in Comparative Examples 1 and 2 respectively was measured. The tests were carried out using distilled water as the test fluid.

The results of Tablet Hardness Test in Test Example 1 and Disintegration Test in Test Example 2 are shown in Table 1.

Table 1 Tablet Hardness and Disintegration Time

| | Hardness (N) | Disintegration Time (min.) |
|---|---|---|
| Tablet 1 | 61.5 | 2.2 |
| Tablet 2 | 46.7 | 23.6 |
| Tablet 3 | 23.6 | 26.5 |

Table 1 shows that the disintegration times of Tablets 2 and 3 having general compositions of the related art are 20 minutes or more and the disintegration times indicate that these tablets are not rapidly disintegrated. In contrast, it is shown that Tablet 1 having the composition of the present invention has higher hardness than those of Tablets 2 and 3, and does rapidly disintegrate in a short period of time.

Test Example 3 (Dissolution Test)

According to the method 2 (puddle method) described in Dissolution Test of The Japanese Pharmacopoeia, Fourteenth Edition, Part I, each of Tablet 1 obtained in Example 1 and Tablets 2 and 3 obtained in Comparative Examples 1 and 2 respectively was applied to the dissolution test. The tests were carried out using 900 mL of an aqueous solution of 2.0 wt% Tween 80 (manufactured by Wako Pure Chemical Industries Ltd.) as the dissolution medium, and the puddle was rotated at the number of 50 rotations per minute. Since the dissolution test started, the amount of Compound 1 dissolved from each tablet was measured by sampling the dissolution medium with passage of time and analyzing by high-performance liquid chromatography (HPLC). The conditions for HPLC analysis are as follows.

< The conditions for HPLC analysis >

Column: Inertsil ODS-2 (manufactured by GL Science); 4.6 mm I.D.×150 mm
Column temperature: 25 °C
Elution solvent: 0.05 mol/L phosphate buffer, pH 6.1/acetonitrile = 3/2
Flow rate of the elution solvent: the flow rate was adjusted so that the retention time of Compound 1 will be about 12 minutes (1.2 mL/min).
Detector: ultraviolet absorptiometer (measurement wavelength: 250 nm)

The results of the dissolution test are shown in Fig.1. Fig.1 shows that Tablets 2 and 3 having general compositions of the related art were not rapidly dissolved. In contrast, the dissolution rate from Tablet 1 having the composition

of the present invention was 80 % or more in 30 minutes. Thus, Compound 1 was rapidly dissolved from Tablet 1.

Test Example 4 (Stability Test of Solid Formulation)

According to the guideline (6 November 1996) for "Photostability Testing of New Drug Substances and Products" in The International Conference on Harmonization of Technical Requirements for Registration of Pharmaceuticals for Human Use (ICH), the stability test on each of Tablet 1 obtained in Example 1 and Tablets 2 to 4 obtained in Comparative Examples 1 to 3 respectively was carried out. Tablets were exposed to the light at an overall illumination of 1,200,000 Lux • hr or more, respectively, using a xenon lamp as the light sources. After exposure, total amounts of decomposition products of Compound 1 in each tablet, which arise from photochemical degradation processes, were measured by sampling and analyzing by HPLC. The conditions for HPLC analysis were the same conditions as shown in Test Example 3.

The results of the stability test are shown in Table 2.

Table 2 Results of Stability Test

| | Tablet 1 | Tablet 2 | Tablet 3 | Tablet 4 |
|---|---|---|---|---|
| Amount (%) of decomposition products of Compound 1 | 0.46 | 1.96 | 1.59 | 1.72 |

Table 2 shows that the amount of the decomposition products of Compound 1 in Tablet 1, which contains microcrystalline cellulose, were clearly suppressed compared with Tablets 2 to 4, which do not contain of microcrystalline cellulose.

As results described above, it was found that the solid formulation of the present invention possess excellent pharmaceutical properties such as excellent hardness, disintegration property and dissolution property, stability and the like.

Brief Description of the Drawings

Fig.1 shows the dissolution properties of Compound 1 from Tablet 1 obtained in Example 1 and Tablets 2 and 3 obtained in Comparative Examples 1 and 2, respectively. The results are plotted with the dissolution ratio (%) of Compound 1 as ordinate, against the dissolution time (min.) as abscissa. The symbols on the graphs have the following meanings, respectively.

-●-: dissolution ratio (%) of Compound 1 from Tablet 1
-○-: dissolution ratio (%) of Compound 1 from Tablet 2
-△-: dissolution ratio (%) of Compound 1 from Tablet 3

Best Mode for Carrying Out the Invention

The present invention is described in more detail in the following Examples. However, these Examples never limit the present invention.

Example 1: Preparation of Tablet 1 (20 mg, core tablet)

According to a prescription described in Table 3 below, a tablet was prepared as follows. Using a fluid-bed granulator with solution spray system (FLOW COATER Type FLO-5, manufactured by Freund Corporation), Compound 1 (230.8 g) obtained in a manner similar to that in Japanese Published Unexamined Patent Application No. 040652/97, lactose (804.2 g, Parmatose, 200 M Lactose, manufactured by DMV International), microcrystalline cellulose (345.0 g, Avicel PH301, manufactured by Asahi Kasei Co., Ltd.) and crospovidone (75.0 g, PVPP, XL-10, manufactured by ISP Japan Ltd.) were mixed. An aqueous solution (375.0 g) of 8.0 wt% polyvinyl alcohol (EG-05, manufactured by Nippon Synthetic Chemical Industry Co., Ltd.) was sprayed on the resulting mixture to prepare a granule. After drying, the resulting granule was milled by a milling machine (Fiole Type F-0 manufactured by Tokujyu Industry Co., Ltd.) to prepare a milled granule. The resulting milled granule (1358.8 g) and magnesium stearate (13.7 g, HyQual® manufactured by Mallinckrodt Inc.) were mixed by a blender (V-blender Type V-10 manufactured by Tokujyu Industry Co., Ltd.) to prepare a granule for tableting. The resulting granule for tableting was tableted by a tableting machine (Correct 12 manufactured by Kikusui Seisakusho Ltd.) to obtain Tablet 1 (tablet weight: 130.0 mg; tablet shape: round shape (7.0 mmΦ)).

Example 2: Preparation of film-coated tablet (10 mg tablet)

According to a prescription described in Table 3 below, an core tablet was prepared as follows. Using a fluid-bed granulator with solution spray system (Gratt® WSG-15, manufactured by Powrex Corporation), Compound 1 (1153.8 g) obtained in a manner similar to that in Japanese,Published Unexamined Patent Application No. 040652/97, lactose (8850.0 g, Parmatose, 200 M Lactose, manufactured by DMV International), microcrystalline cellulose (3796.2 g, Avicel PH301, manufactured by Asahi Kasei Co., Ltd.) and crospovidone (750.0 g, PVPP, XL-10, manufactured by ISP Japan Ltd.) were mixed. An aqueous solution (3750.0 g) of 8.0 wt% polyvinyl alcohol (EG-05, manufactured by Nippon Synthetic Chemical Industry Co., Ltd.) was sprayed on the resulting mixture to prepare a granule. After drying, the resulting granule was milled by a milling machine (Fiole Type F-0 manufactured by Tokuj yu Industry Co., Ltd.) to prepare a milled granule. The resulting milled granule (6930.0 g) and magnesium stearate (70.0g, HyQual® manufactured by Mallinckrodt) were mixed by a blender (Type TBM-25 manufactured by Tokujyu Industry Co., Ltd.) to prepare a granule for tableting. The resulting granule for tableting was tableted by a tableting machine (Correct 12 manufactured by Kikusui Seisakusho Ltd.) to obtain the core tablet (tablet weight: 130.0 mg; tablet shape: round shape (7.0 mm Φ)). A coating composition, which was prepared according to a formulation described in Table 2 below, was dissolved and dispersed in distilled water to prepare a coating solution having a solid content of 10 wt%. The core tablets obtained above (1000.0 g) were coated with a coated layer, in which the coated layer is at 5 parts by weight per 100 parts by weight of the core tablet in a dry state, by Hi-Coater (HCT-30 manufactured by Freund Corporation) to obtain a film-coated tablet.

Comparative Example 1: Preparation of Tablet 2 (20 mg, core tablet)

According to a prescription described in Table 3 below, a tablet was prepared as follows. Using an agitating granulation machine (Type VG-05, manufactured by Powrex Corporation), Compound 1 (100.0 g) obtained in a manner similar to that in Japanese Published Unexamined Patent Application No. 040652/97, lactose (717.0 g, Parmatose, 200 M Lactose, manufactured by DMV International), potato starch (150.0 g, manufactured by Nippon Starch Chemical Co., Ltd.) and hydroxypropylcellulose (20.0 g, HPC-L, manufactured by Nippon Soda Co., Ltd.) were mixed, and an aqueous solution (130.0 g) of 7.7 wt% hydroxypropylcellulose (HPC-L, manufactured by Nippon Soda Co., Ltd.) was sprayed thereon followed by kneading the mixture. After drying, the resulting granule was milled by a milling machine (Comil® 197S, manufactured by Powrex Corporation) to prepare a milled granule.

The resulting milled granule (802.1 g) and magnesium stearate (2.4 g, HyQual® manufactured by Mallinckrodt Inc.) were mixed by a blender (V-blender Type V-10 manufactured by Tokuj yu Industry Co., Ltd.) to prepare a granule for tableting. The resulting granule for tableting was tableted by a tableting machine (Collect 12 manufactured by Kikusui Seisakusho Ltd.) to obtain Tablet 2 (tablet weight: 200.0 mg; tablet shape: round shape (8.0 mm Φ)).

Comparative Example 2: Preparation of Tablet 3 (20 mg, core tablet)

According to a prescription described in Table 3 below, the tablet was prepared as follows. Using an agitating granulation machine (Type VG-05, manufactured by Powrex Corporation), Compound 1 (200.0 g) obtained in a manner similar to that in Japanese Published Unexamined Patent Application No. 040652/97, lactose (734.0 g, Parmatose, 200 M Lactose, manufactured by DMV International), potato starch (300.0 g, manufactured by Nippon Starch Chemical Co., Ltd.) and hydroxypropylcellulose (50.0 g, HPC-L, manufactured by Nippon Soda Co., Ltd.) were mixed, and an aqueous solution (200.0 g) of 5.0 wt% hydroxypropylcellulose (HPC-L, manufactured by Nippon Soda Co., Ltd.) was sprayed thereon followed by kneading the mixture. After drying, the resulting granule was milled by a millingmachine (Comil® 197S, manufactured by Powrex Corporation) to prepare a milled granule.

The resulting milled granule (1196.6 g) and magnesium stearate (7.2 g, HyQual® manufactured by Mallinckrodt) were mixed by a blender (V-blender Type V-10 manufactured by Tokuj yu Industry Co., Ltd.) to prepare a granule for tableting. The resulting granule for tableting was tableted by a tableting machine (Correct 12 manufactured by Kikusui Seisakusho Ltd.) to obtain Tablet 3 (tablet weight: 130.0 mg; tablet shape: round shape (7.0 mm Φ)).

Comparative Example 3: Preparation of Tablet 4 (20 mg, core tablet)

According to a prescription described in Table 3 below, the tablet was prepared as follows. Using fluid-bed granulator with solution spray system (Type FLO-2, manufactured by Freund Industrial Co., Ltd.), Compound 1 (200.0 g) obtained in a manner similar to that in Japanese Published Unexamined Patent Application No. 040652/97, lactose (697.0 g, Parmatose, 200 M Lactose, manufactured by DMV International), partialalpha starch (299.0 g, Starch 1500 G, manufactured by Colorcon) and crospovidone (65.0 g, PVPP, XL-10, manufactured by ISP Japan Ltd.) were mixed. An aqueous solution (325.0 g) of 8.0 wt% polyvinyl alcohol (EG-05 manufactured by Nippon Synthetic Chemical Industry Co., Ltd.) was sprayed on the resulting mixture to prepare a granule. After drying, the resulting granule was milled by a milling machine (Comil® 197S, manufactured by Powrex Corporation) to prepare a milled granule. The resulting milled granule (990.0 g) and magnesium stearate (10.0 g, HyQual® manufactured by Mallinckrodt) were mixed by a blender

(Type TBM-8 manufactured by Tokuj yu Industry Co., Ltd.) to prepare a granule for tableting. The resulting granule for tableting was tableted by a tableting machine (Correct 12 manufactured by Kikusui Seisakusho Ltd.) to obtain Tablet 4 (tablet weight: 130.0 mg; tablet shape: round shape (7.0 mm Φ)).

Table 3 Prescriptions for Examples and Comparative Examples (mg)

| | Example 1 | Example 2 | Comparative Example 1 | Comparative Example 2 | Comparative Example 3 |
|---|---|---|---|---|---|
| (Uncoated tablet) | | | | | |
| Compound 1 | 20.0 | 10.0 | 20.0 | 20.0 | 20.0 |
| Lactose | 69.7 | 76.7 | 143.4 | 73.4 | 69.7 |
| Potato starch | | | 30.0 | 30.0 | |
| Partial pregelatinized starch | | | | | 29.9 |
| Microcrystalline cellulose | 29.9 | 32.9 | | | |
| Crospovidone | 6.5 | 6.5 | | | 6.5 |
| Polyvinyl alcohol | 2.6 | 2.6 | | | 2.6 |
| HPC-L | | | 6.0 | 6.0 | |
| Magnesium stearate | 1.3 | 1.3 | 0.6 | 0.6 | 1.3 |
| (Coating composition*) | | | | | |
| HPMC 2910 | | 3.51 | | | |
| Lactose | | 1.43 | | | |
| Macrogol 4000 | | 0.52 | | | |
| Triacetin | | 0.39 | | | |
| Talc | | 0.65 | | | |
| Total | 130.0 | 136.5 | 200.0 | 130.0 | 130.0 |

* HPMC 2910 (TC-5E; manufactured by Shin-Etsu Chemical Co., Ltd.), Lactose (Parmatose, 200M Lactose; manufactured by DMV International), Macrogol 4000 (manufactured by NOF Corporation), Triacetin (manufactured by Yuki Gosei Kogyo Co., Ltd.), Talc (Risuburan; manufactured by Kihara Kasei)

Industrial Applicability

The present invention provides a solid formulation comprising Compound (I) or a pharmaceutically acceptable salt thereof, which possesses excellent pharmaceutical properties (for example, in hardness, disintegration property, dissolution property, stability or the like).

## Claims

**1.** A solid formulation comprising a xanthine derivative represented by formula (I)

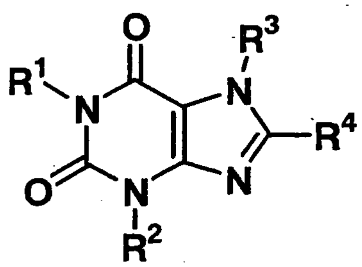

(I)

[wherein $R^1$, $R^2$ and $R^3$ may be the same or different and each represents a hydrogen atom, lower alkyl, lower alkenyl or lower alkynyl; and $R^4$ represents cycloalkyl, $-(CH_2)_n-R^5$ (in which $R^5$ represents substituted or unsubstituted aryl or a substituted or unsubstituted heterocyclic group; and n represents an integer of 0 to 4) or formula (II)

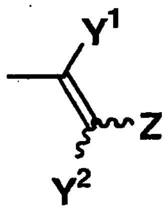

(II)

(in which $Y^1$ and $Y^2$ may be the same or different and each represents a hydrogen atom, halogen or lower alkyl; and Z represents substituted or unsubstituted aryl or a substituted or unsubstituted heterocyclic group)] or a pharmaceutically acceptable salt thereof, and microcrystalline cellulose.

2. The solid formulation according to claim 1, wherein $R^4$ is formula (II)

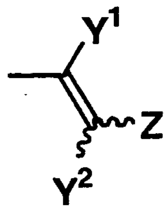

(II)

(in which $Y^1$, $Y^2$ and Z have the same meanings as described above, respectively).

3. The solid formulation according to claim 2, wherein $Y^1$ and $Y^2$ each are a hydrogen atom.

4. The solid formulation according to claim 2 or 3, wherein Z is substituted or unsubstituted aryl or formula (III)

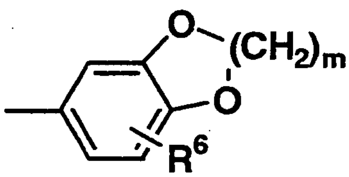

(III)

(in which $R^6$ represents a hydrogen atom, hydroxy, lower alkyl, lower alkoxy, halogen, nitro or amino; and m represents an integer of 1 to 3).

5. A solid formulation comprising (E)-8-(3,4-dimethoxystyryl)-1,3-diethyl-7-methyl-3,7-dihydro -1H-purine-2,6-dione represented by formula (IA)

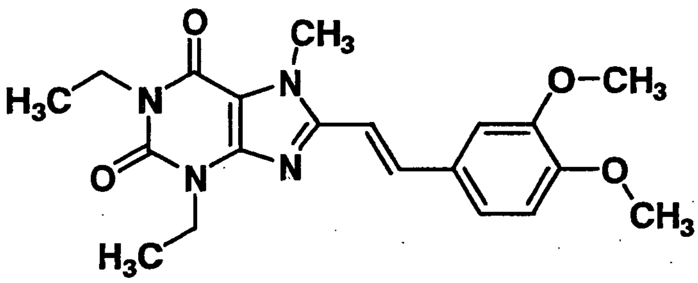

(IA)

or a pharmaceutically acceptable salt thereof, and microcrystalline cellulose.

6. The solid formulation according to any one of claims 1 to 5, which contains a sugar.

7. The solid formulation according to claim 6, wherein the sugar content is 1.0 to 9.0 parts by weight per 1.0 part by weight of microcrystalline cellulose.

8. The solid formulation according to claim 6, wherein the sugar content is 1.0 to 5.0 parts' by weight per 1.0 part by weight of microcrystalline cellulose.

9. The solid formulation according to claim 6, wherein the sugar content is 1.5 to 3.0 parts by weight per 1.0 part by weight of microcrystalline cellulose.

10. The solid formulation according to any one of claims 6 to 9, wherein the sugar is lactose, sucrose, glucose, cyclodextrin or mannitol.

11. The solid formulation according to any one of claims 6 to 9, wherein the sugar is lactose.

12. The solid formulation according to any one of claims 1 to 11, which contains a binder.

13. The solid formulation according to claim 12, wherein the binder is hydroxypropylcellulose, hydroxypropylmethylcellulose, polyvinylpyrrolidone or polyvinyl alcohol.

14. The solid formulation according to claim 12, wherein the binder is polyvinyl alcohol.

15. The solid formulation according to any one of claims 12 to 14, wherein the binder content is 0.1 to 10.0 % of the total weight of the solid formulation.

16. The solid formulation according to any one of claims 1 to 15, which contains a disintegrator.

17. The solid formulation according to claim 16, wherein the disintegrator is crospovidone, croscarmellose sodium, low substituted hydroxypropylcellulose, carmellose calcium or sodium starch glycolate.

18. The solid formulation according to claim 16, wherein the disintegrator is crospovidone.

19. The solid formulation according to any one of claims 16 to 18, wherein the disintegrator content is 0.5 to 20.0 % of the total weight of the solid formulation.

20. The solid formulation according to any one of claims 1 to 19, wherein a dosage form of the solid formulation is a tablet.

21. The solid formulation according to any one of claims 1 to 20, which is a film-coated solid formulation.

22. A method for stabilizing a xanthine derivative represented by formula (I)

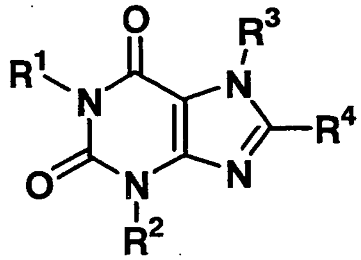

(I)

(wherein $R^1$, $R^2$, $R^3$ and $R^4$ have the same meanings as described above, respectively) or a pharmaceutically acceptable salt thereof in a solid formulation comprising the xanthine derivative or the pharmaceutically acceptable salt thereof, which comprises allowing microcrystalline cellulose to exist in the solid formulation.

FIG.1

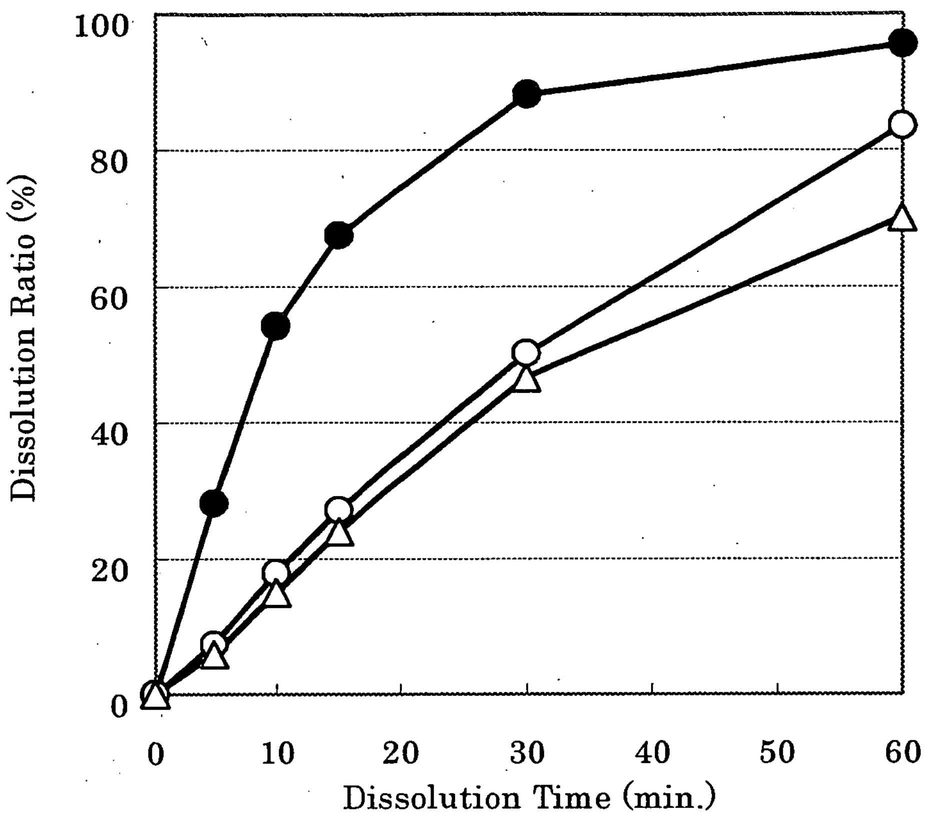

100
80
60
40
20
0
Dissolution Ratio (%)
0
10
20
30
40
50
60
Dissolution Time (min.)

**INTERNATIONAL SEARCH REPORT**

International application No.
PCT/JP2004/010530

A. CLASSIFICATION OF SUBJECT MATTER
Int.Cl[7] A61K31/522, 9/20, 47/38, A61P25/16, 25/24, 25/28

According to International Patent Classification (IPC) or to both national classification and IPC

B. FIELDS SEARCHED

Minimum documentation searched (classification system followed by classification symbols)
Int.Cl[7] A61K31/522, 9/20, 47/38, A61P25/16, 25/24, 25/28

Documentation searched other than minimum documentation to the extent that such documents are included in the fields searched

Electronic data base consulted during the international search (name of data base and, where practicable, search terms used)
CA(STN), MEDLINE(STN)

C. DOCUMENTS CONSIDERED TO BE RELEVANT

| Category* | Citation of document, with indication, where appropriate, of the relevant passages | Relevant to claim No. |
|---|---|---|
| Y | JP 06-211856 A (Kyowa Hakko Kogyo Co., Ltd.), 02 August, 1994 (02.08.94), Full text & US 5484920 A & US 5587378 A | 1-22 |
| Y | JP 2000-273038 A (Taisho Pharmaceutical Co., Ltd.), 03 October, 2000 (03.10.00), Full text; particularly, Par. No. [0018] (Family: none) | 1-22 |
| Y | JP 2002-255797 A (Eisai Co., Ltd.), 11 September, 2002 (11.09.02), Full text; particularly, Par. No. [0064] (Family: none) | 1-22 |

[X] Further documents are listed in the continuation of Box C. [ ] See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 28 September, 2004 (28.09.04) | 12 October, 2004 (12.10.04) |
| Name and mailing address of the ISA/ Japanese Patent Office | Authorized officer |
| Facsimile No. | Telephone No. |

Form PCT/ISA/210 (second sheet) (January 2004)

**INTERNATIONAL SEARCH REPORT**

International application No.
PCT/JP2004/010530

C (Continuation). DOCUMENTS CONSIDERED TO BE RELEVANT

| Category* | Citation of document, with indication, where appropriate, of the relevant passages | Relevant to claim No. |
|---|---|---|
| Y | JP 3-258730 A (Asahi Chemical Industry Co., Ltd.), 19 November, 1991 (19.11.91), Full text, particularly, page 3, upper left column, lines 1 to 4 (Family: none) | 1-22 |
| A | Yasutaka WADA et al., "Funtai no Asshukusei ni Taisuru Fuchaku Seibun no Eikyo -Kyushitsu Funmatsu o Mochiita Seizoho no Sekkei to sono Kiso Kenkyu-", Journal of Pharmaceutical Science and Technology, Japan, Vol.50, No.2, 1990, pages 215 to 224 | 1-22 |

Form PCT/ISA/210 (continuation of second sheet) (January 2004)